# EUROPEAN PATENT SPECIFICATION

(11) **EP 2 328 540 B1**
(45) Date of publication and mention of the grant of the patent: **29.05.2013**
(21) Application number: 09787201.4
(22) Date of filing: 15.09.2009
(51) Int. Cl.: A61K 8/06, A61K 8/31, A61K 8/34, A61K 8/39, A61Q 1/02

(54) **EMULSION-TYPE COMPOSITIONS COMPRISING AT LEAST ONE VOLATILE LINEAR ALKANE**
ZUSAMMENSETZUNGEN VOM EMULSIONSTYP MIT MINDESTENS EINEM FLÜCHTIGEN LINEAREN ALKAN
COMPOSITIONS DE TYPE ÉMULSION COMPRENANT AU MOINS UN ALCANE LINÉAIRE VOLATILE

(30) Priority: 25.09.2008 FR 0856461; 17.10.2008 US 106163 P
(43) Date of publication of application: 08.06.2011
(73) Proprietor: L'Oréal, 75008 Paris (FR)
(72) Inventor: CLAVEL, Euriel, F-75013 Paris (FR); ARNAUD, Pascal, F-94240 L'hay Les Roses (FR); ESCARRACHI-FEVRE, Paloma, F-94130 Nogent Sur Marne (FR)
(74) Representative: Le Coupanec, Pascale A.M.P.
(86) International application number: PCT/IB2009/054031
(87) International publication number: WO 2010/035169

(56) References cited:
- AU-B2- 522 120
- DE-A1-102006 053 360
- US-B1- 7 214 717

## Description

The present invention relates to emulsion-type compositions dedicated to making up and/or caring for keratin materials, in particular the skin and the lips.

In particular, the present invention relates to emulsion-type cosmetic and/or dermatological compositions comprising a fatty continuous phase, preferably of water-in-oil emulsion type.

In the cosmetics and/or dermatological field, emulsions, especially water-in-oil emulsions, are particularly appreciated in the field of foundations, sun products or moisturizing creams, from the viewpoint of their cosmetic properties, in particular from the viewpoint of their comfort on application.

The comfort on application is in particular reflected by a lack of tautness, of feelings of drying out and/or of tacky and/or greasy sensations. For obvious reasons, these properties, in the same way as the stability of the corresponding emulsions, are closely dependent on the nature of the compounds that go to make up the formulation of a composition and the galenic formulation thereof.

Now, consumers are increasingly searching for cosmetic products made up, completely or partly, of natural constituents or constituents of natural origin.

The term "natural compound" is intended to mean a compound that is obtained directly from the earth or the soil, or from plants or animals, *via,* as appropriate, one or more physical processes, for instance grinding, refining, distillation, purification or filtration.

The term compounds "of natural origin" is intended to mean a natural compound having undergone one or more secondary chemical or industrial treatments generating modifications that do not affect the essential qualities of this compound and/or a compound comprising predominantly natural constituents which may or may not have undergone transformations, as indicated above.

By way of nonlimiting example of a secondary chemical or industrial treatment generating modifications that do not affect the essential qualities of a natural compound, mention may be made of those authorized by control organizations such as Ecocert (System of reference for biological and ecological cosmetic products, January 2003) or defined in the manuals recognized in the field, such as "Cosmetics and Toiletries Magazine", 2005, Vol. 120, 9:10.

For obvious reasons, this expectation by users cannot be taken into account in the production of conventional emulsions without it raising difficulties with regard in particular to incompatibilities between these "natural" compounds and those conventionally selected, and which are prejudicial to the stability of the emulsions.

Thus, the use of polyglyceryl polyricinoleates which constitute, as described in patent DE 44 09 569, a preferred class of surfactants of natural origin in so far as they result from an esterification reaction between polyglycerols and acids of plant origin, can impair the stability of the compositions containing them, resulting in particular in problems of coalescence.

To overcome this drawback, it has been proposed to introduce into the aqueous phase of these emulsions high levels of polyols, so as to increase the stability of the compositions.

US 6,013,255 and WO 2008/055692 describe the use of a polyol in emulsions comprising polyglyceryl polyricinoleate.

However, this solution may lead to a tacky sensation on application or after applying make-up, and is therefore not always satisfactory in terms of comfort on application.

DE 10 2006 053360 A1 (EVONIK STOCKHAUSEN GMBH [DE]) 15 May 2008 (2008-05-15) discloses (ex. K) an emulsion-type cosmetic composition comprising a fatty continuous phase (W/O, § [0043]), said composition comprising at least:
(A) one polyglyceryl polyricinoleate: Crester PR
(B) one polyol: sorbitol, glycerol, PG
(C) one volatile alkane: isohexadecane.

Consequently, there remains the need to provide emulsion-type compositions having satisfactory, or even improved, properties, both in cosmetic terms and in terms of stability.

There also exists a need to have stable, emulsion-type compositions, in particular for caring for and/or making up keratin materials, for which the comfort on application is maintained, or even improved.

In particular, there exists a need to have emulsion-type compositions, in particular comprising a fatty continuous phase, having little or no greasy and/or tacky nature.

The object of the present invention is to meet these needs.

More specifically, according to one of its first aspects, the invention relates to an emulsion-type cosmetic and/or dermatological composition comprising a fatty continuous phase, preferably of water-in-oil emulsion type, said composition comprising at least one polyglyceryl polyricinoleate, at least one polyol and at least one volatile linear alkane.

Unexpectedly, as illustrated by the examples, the inventors have observed that the combination of at least one specific volatile linear alkane, for example n-undecane or n-tridecane, or a mixture thereof, with at least one polyglyceryl polyricinoleate and at least one polyol makes it possible to formulate emulsions comprising a fatty continuous phase, in particular of water-in-oil emulsion type, having satisfactory, or even improved, properties, both in terms of comfort on application and in terms of stability.

An emulsion according to the invention comprising a fatty continuous phase may in particular be a water-in-oil (W/O) or multiple (O/W/O) emulsion.

Preferably, an emulsion according to the invention is a water-in-oil emulsion.

According to one embodiment variant, the volatile linear alkane is an alkane of plant origin.

For the purpose of the invention, the term "compound of plant origin" is intended to mean a compound derived from a plant or having undergone one or more chemical modifications, for example by organic synthesis reaction.

For the purpose of the invention, the term "plant compound" is intended to mean a compound immediately derived from a plant without having undergone chemical modification.

Advantageously, the compositions of the invention exhibit little, or even no, tacky and/or greasy effect on application.

According to another of its aspects, the present invention relates to the use of at least one volatile linear alkane in an emulsion-type composition comprising a fatty continuous phase, preferably of water-in-oil emulsion type, said composition comprising at least one polyglyceryl polyricinoleate and at least one polyol, for conferring on said composition improved comfort on application and/or improved stability.

A subject of the present invention is also a cosmetic method for making up and/or caring for keratin materials, comprising at least the application, to said materials, of at least one layer of a composition according to the invention.

According to another of its aspects, a method of the invention makes it possible in particular to confer comfort on application and/or improved stability.

According to one embodiment variant, a cosmetic make-up method of the invention may also be carried out on a synthetic support.

The present invention relates to cosmetic compositions which may be in the form of a dermatological composition or of a composition for caring for keratin materials, in particular the skin or the lips, or else in the form of an antisun composition. It may then be in a noncoloured form, optionally containing cosmetic or dermatological active agents. It may then be used as a care base for keratin materials, in particular the skin or the lips.

A composition of the invention may also be in the form of a coloured product for making up keratin materials, in particular for making up the skin or the lips, such as a foundation, a blusher, a face powder or an eyeshadow, a skin-colouring product, a concealer product, a lipstick or a lip balm, or else for making up the eyelashes or the eyebrows, such as a mascara.

According to the invention, the term "keratin materials" is intended to denote the skin of the body or the mucous membranes, for example the face or the lips, and also body hair and head hair, and in particular the eyelashes.

In addition to the compounds mentioned above, the additional compounds or additives which go to make up the formulation of the invention are preferably natural or of natural origin.

For the purpose of the invention, the term "synthetic or artificial compound" is intended to mean a compound which does not comply with the definitions of the natural compounds or compounds of natural origin, as given above.

According to one embodiment, a composition of the invention is substantially constituted of natural compounds or compounds of natural origin.

### STABILITY

According to the invention, the term "stable emulsion" is intended to mean a homogeneous and regular emulsion (water-in-oil) which does not undergo phase separation (separation of the aqueous phase and of the fatty phase) and which does not release oil.

The stability of a composition of the invention may be evaluated by means of the following protocols.

The stability of the compositions in accordance with the invention may, for example, be evaluated by monitoring the change in size of the globules of the aqueous phase over time.

When the average size of the globules of a composition increases over time, this means that it is subject to problems of coalescence, resulting in a phenomenon of heterogeneity and reflecting instability of the composition.

This monitoring of the change in globule size can be carried out by means of particle-size measurements.

By way of example of a protocol that is suitable for the invention, mention may be made of the following protocol.

Particle-size measurements are carried out on samples of 25 ml of the compositions to be analysed, on the actual day of production thereof, and also after 14 days and 30 days of storage at 45°C.

These measurements can be carried out using a Mastersizer 2000 particle sizer from Malvern Instruments, which makes it possible to determine the particle size distribution by light diffraction. The value of the volume-average diameter, noted D [4,3] and expressed in µm, can then be obtained from the particle sizer.

The stability of an emulsion may also be evaluated by means of a visual assessment. For example, the stability of a composition may be evaluated by observation of the size of the sedimentation occurring for a sample of the composition, after storage for two months at 45°C.

Alternatively, the stability of a composition may be evaluated by observation of a sample of said composition after storage at ambient temperature for 48 hours, under a microscope, at a magnification of X100.

The microscopic appearance of said composition should remain close to the initial appearance. In particular, no degradation of the emulsion (coarser emulsion base, coalescence as indicated by the presence of numerous large drops, modification of the preparation edges, presence of crystals) should be observed.

Alternatively, the stability of a composition may also be evaluated according to any protocol known to those skilled in the art.

### VOLATILE LINEAR ALKANES

According to one embodiment, a volatile linear alkane suitable for the invention may have a flashpoint in the range of from 70 to 120°C, and more particularly from 80 to 100°C, and especially of approximately 89°C.

A volatile linear alkane suitable for the invention is liquid at ambient temperature (approximately 25°C).

According to one embodiment, an alkane suitable for the invention may be a volatile linear alkane containing from 7 to 17 carbon atoms, in particular from 9 to 15 carbon atoms, and more particularly from 11 to 13 carbon atoms.

A volatile linear alkane suitable for the invention may advantageously be of plant origin.

Such an alkane may be obtained, directly or in several steps, from a plant starting material such as an oil, a butter, a wax, etc.

By way of examples of an alkane suitable for the invention, mention may be made of the alkanes described in the Cognis Patent Application WO 2007/068371.

These alkanes are obtained from fatty alcohols, which are themselves obtained from coconut oil or palm oil.

By way of example of a linear alkane suitable for the invention, mention may be made of n-nonane (C9), n-decane (C10), n-undecane (C11), n-dodecane (C12), n-tridecane (C13), n-tetradecane (C14), n-pentadecane (C15), n-hexadecane (C16) and n-heptadecane (C17), and mixtures thereof, and in particular the mixture of n-undecane (C11) and n-tridecane (C13) sold under the reference Cetiol UT by the company Cognis.

According to one particular embodiment, a volatile linear alkane suitable for the invention may be selected from n-nonane, n-undecane, n-dodecane, n-tridecane and n-heptadecane, and mixtures thereof.

More particularly, a volatile linear alkane suitable for the invention may be used in the form of an n-undecane/n-tridecane mixture.

Preferably, in such a mixture, the n-undecane: n-tridecane weight ratio may be 50:50 to 90:10, preferably ranging from 60:40 to 80:20, preferably ranging from 65:35 to 75:25.

In particular, a composition according to the invention may comprise an n-undecane: n-tridecane mixture in a weight ratio of 70:30.

According to one particular embodiment, a volatile hydrocarbon-based solvent suitable for the invention may have an evaporation rate of less than or equal to 0.13 mg/cm²/min.

According to one embodiment, a volatile hydrocarbon-based solvent suitable for the invention may have an evaporation rate in the range of from 0.05 to 0.13 mg/cm²/min, in particular from 0.08 to 0.12 mg/cm²/min, and more particularly from 0.1 to 0.12 mg/cm²/min.

The volatility of a volatile hydrocarbon-based solvent in accordance with the invention may be evaluated in particular by means of the protocol described in WO 06/013413, and more particularly by means of the protocol described below.

15 g of volatile hydrocarbon-based solvent are placed in a crystallizing dish (diameter: 7 cm) which is placed on a balance located in a chamber of approximately 0.3 m³ which is regulated in temperature (25°C) and in hygrometry (relative humidity 50%).

The liquid is allowed to evaporate freely, without stirring, with ventilation provided by a fan (Papst-Motoren, reference 8550 N, rotating at 2700 rpm) placed in a vertical position above the crystallizing dish containing the volatile hydrocarbon-based solvent, the blades being directed towards the crystallizing dish, at a distance of 20 cm from the base of the crystallizing dish.

At regular intervals of time, the mass of volatile hydrocarbon-based solvent remaining in the crystallizing dish is measured.

Evaporation rates are expressed in mg of volatile hydrocarbon-based solvent evaporated per unit surface area (cm²) per unit time (minute).

The volatile hydrocarbon-based solvent according to the invention has an evaporation rate of less than or equal to 0.13 mg/cm²/min. This therefore corresponds to an amount of evaporated solvent of less than or equal to 3.9 mg/cm² in 30 minutes.

A composition of the invention may comprise from 1% to 50% by weight of volatile linear alkane(s), in particular from 5% to 35% by weight of volatile linear alkane(s), and more particularly from 8% to 25% by weight of volatile linear alkane(s), relative to the total weight of the composition.

### POLYGLYCERYL POLYRICINOLEATES

A composition according to the invention comprises at least one surfactant selected from polyglyceryl polyricinoleates.

For the purpose of the invention, the term "polyglyceryl polyricinoleate" denotes an ester resulting from the esterification of one or more polyglycerols with at least one polyricinoleic acid.

A polyglycerol suitable for the invention may be selected from the compounds of general formula (I) below: 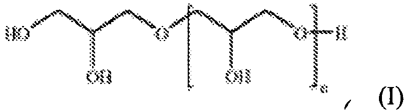 in which n represents an integer between 1 and 11, and in particular between 1 and 7.

A polyricinoleic acid suitable for the invention may be selected from the compounds of general formula (II) below: 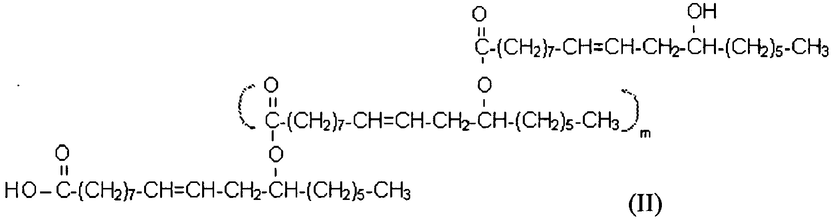 in which m represents an integer between 0 and 10, in particular between 1 and 8, and more particularly between 1 and 5.

A polyglyceryl polyricinoleate suitable for the invention may be a total or partial ester.

Preferably, a polyglyceryl polyricinoleate suitable for the invention is a partial ester.

For the purpose of the invention, the term "partial ester" is intended to denote a compound in which not all the -OH groups of the polyglycerol units have been esterified with polyricinoleic acid, in other words, a polyglyceryl polyricinoleate compound comprising at least one free -OH group on the polyglycerolated chain.

By way of example, a polyglyceryl polyricinoleate suitable for the invention may be a compound of general formula (III) below: 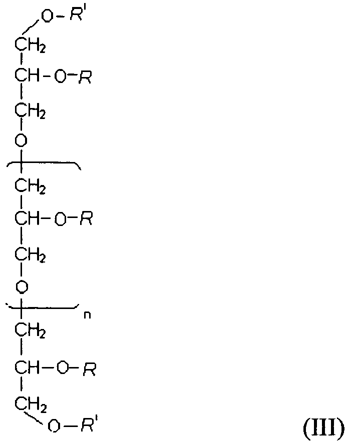 in which R and R' represent, indifferently, radicals selected from a hydrogen atom or a polyricinoleate chain, with the proviso that at least one of these radicals R or R' is a polyricinoleate chain.

Preferably, at least one of the R or R' groups of the polyglycerolated chain is a hydrogen atom.

According to one embodiment, a polyglyceryl polyricinoleate surfactant suitable for the invention may have an HLB (Hydrophilic-Lipophilic Balance) of between 1 and 10, and more particularly between 3 and 8.

By way of example of polyglyceryl polyricinoleate(s) suitable for the invention, mention may in particular be made of polyglyceryl-3 polyricinoleates, in particular sold by the company Karlshamns under the name Akoline PGPR or by the company Stéarineries Dubois Fils under the name DUB PGPR or by the company Dr. Straetmans under the name Dermofeel, or by the company Croda under the name Crester PR or else by the company Sasol under the name Imwitor 600; the polygyceryl-5 polyricinoleates sold by the company Taiyo Kagaku Co. Ltd under the name Sunsoft NO.818R; the polyglyceryl-6 polyricinoleates sold by the company Nikko Chemicals Co. Ltd under the name Hexaglyn PR-15 or by the company Sakamoto Yakuhin Kogyo Co. Ltd under the name S-Face CR-1001; and the polyglyceryl-10 polyricinoleates sold by the company Nikko-Chemicals Co. Ltd under the name Decaglyn PR-20.

According to one embodiment, mixtures of these compounds may be used.

More particularly, a polyglyceryl polyricinoleate suitable for the invention may be selected from polyglyceryl-3 polyricinoleate, and polyglyceryl-6 polyricinoleate, and mixtures thereof.

The proportion of surfactant present in a composition of the invention is shown in such a way as to effectively stabilize the emulsions more particularly considered according to the invention, i.e., in particular, of O/W or O/W/O type. The choice is part of the competence of those skilled in the art.

Thus, a composition of the invention may comprise from 2% to 10% by weight of polyglyceryl polyricinoleate(s), in particular from 3% to 8% by weight of polyglyceryl polyricinoleate(s), and more particularly from 4% to 7% by weight of polyglyceryl polyricinoleate(s), relative to the total weight of the composition.

### POLYOLS

A composition according to the invention comprises at least one polyol.

For the purpose of the present invention, the term "polyol" should be understood to mean any organic molecule comprising at least two free hydroxyl groups.

A polyol suitable for the invention may be a compound such as a saturated or unsaturated, linear, branched or cyclic alkyl bearing, on the alkyl chain, at least two -OH functions, in particular at least three -OH functions, and more particularly at least four -OH functions.

The polyols advantageously suitable for the formulation of the cosmetic compositions according to the present invention are those having, in particular, from 2 to 16 carbon atoms, preferably 3 to 8 carbon atoms.

According to one particular embodiment of the invention, the polyol is a compound such as a saturated or unsaturated, linear, branched or cyclic alkyl bearing, on the alkyl chain, at least four -OH functions.

According to another embodiment, a polyol suitable for the invention may be advantageously selected from polyethylene glycols.

According to one embodiment, a composition of the invention may comprise a mixture of polyols.

Advantageously, the polyol may, for example, be selected from propylene glycol, 1,3-propanediol, butylene glycol, isoprene glycol, pentylene glycol, hexylene glycol, glycerol, polyglycerols, such as glycerol oligomers, for instance diglycerol, erythritol, arabitol, adonitol, sorbitol, dulcitol, glucose, fructose, xylose, trehalose, sucrose, maltose, saccharose and lactose, and mixtures thereof.

According to one preferred embodiment of the invention, said polyol is sorbitol.

According to one embodiment, a mixture of polyol(s) suitable for the invention may advantageously comprise at least one compound such as a saturated or unsaturated, linear, branched or cyclic alkyl bearing, on the alkyl chain, at least four -OH functions, and preferably at least sorbitol.

A polyol suitable for the invention may advantageously be natural or of natural origin.

A composition of the invention may comprise from 2% to 25% by weight of polyol(s), in particular from 5% to 20% by weight of polyol(s), and more particularly from 6% to 15% by weight ofpolyol(s), relative to the total weight of the composition.

According to one particular embodiment of the invention, the volatile linear alkane(s) is (are) present in the composition, by weight relative to the total weight of the polyol(s) and of the polyglyceryl polyricinoleate(s), in a weight ratio ranging from 0.25 to 2.

In particular, the volatile linear alkane(s) is (are) present in the composition, by weight relative to the total weight of the polyol(s) and of the polyglyceryl polyricinoleate(s), in a weight ratio ranging from 0.3 to 1.5.

Preferably, the volatile linear alkane(s) is (are) present in the composition, by weight relative to the total weight of the polyol(s) and of the polyglyceryl polyricinoleate(s), in a weight ratio ranging from 0.5 to 1.3.

### PHYSIOLOGICALLY ACCEPTABLE MEDIUM

In addition to the compounds indicated above, a composition according to the invention comprises a physiologically acceptable medium.

The term "physiologically acceptable medium" is intended to denote a medium which is particularly suitable for the application of a composition of the invention to keratin materials, in particular the skin and the lips.

The physiologically acceptable medium is generally suitable for the nature of the support to which the composition should be applied, and also for the way in which the composition should be packaged.

### Aqueous phase

A composition of the invention may comprise water in a content ranging from 5% to 80%, and more particularly from 20% to 60% by weight, relative to the total weight of the composition.

Water suitable for the invention may, for example, be a demineralized water, a water from a natural source, such as La Roche-Posay water, a floral water such as cornflower water and/or a spring water.

According to one embodiment, a composition of the invention may also comprise at least one water-miscible organic solvent.

The water-miscible organic solvent(s) suitable for the invention may be selected from C₁₋₈, and in particular C₁₋₅, monoalcohols, in particular ethanol, isopropanol, tert-butanol, n-butanol, the polyols as described above, and mixtures thereof.

A composition of the invention may also comprise at least one salt, for example sodium chloride, magnesium chloride and magnesium sulphate.

A composition of the invention may comprise from 0.05% to 1.5%, in particular from 0.1% to 1.0%, and more particularly from 0.15% to 0.8% by weight of salts, relative to the total weight of the composition.

### Liquid fatty phase

A cosmetic composition in accordance with the present invention may comprise at least one liquid and/or solid fatty phase, and in particular at least one oil as mentioned hereinafter.

The term "oil" is intended to mean any fatty substance in liquid form at ambient temperature (20 - 25°C) and at atmospheric pressure.

A composition of the invention may comprise a liquid fatty phase in a content ranging from 5% to 95%, in particular from 10% to 80%, in particular from 15% to 70%, and more particularly from 20% to 65% by weight, relative to the total weight of the composition.

The oily phase suitable for the preparation of the cosmetic compositions according to the invention may comprise hydrocarbon-based, silicone, fluoro or non-fluoro oils, or mixtures thereof.

The oils may be volatile or non-volatile.

They may be of animal, plant, mineral or synthetic origin. According to one embodiment variant, oils of plant origin are preferred.

For the purpose of the present invention, the term "volatile oil" is intended to mean an oil (or nonaqueous medium) capable of evaporating on contact with the skin in less than one hour, at ambient temperature and at atmospheric pressure. The volatile oil is a volatile cosmetic oil which is liquid at ambient temperature, having in particular a non-zero vapour pressure, at ambient temperature and at atmospheric pressure, in particular having a vapour pressure ranging from 0.13 Pa to 40 000 Pa (10⁻³ to 300 mmHg), and preferably ranging from 1.3 Pa to 13 000 Pa (0.01 to 100 mmHg), and preferentially ranging from 1.3 Pa to 1300 Pa (0.01 to 10 mmHg).

For the purpose of the present invention, the term "non-volatile oil" is intended to mean an oil having a vapour pressure of less than 0.13 Pa.

For the purpose of the present invention, the term "silicone oil" is intended to mean an oil comprising at least one silicon atom, and in particular at least one Si-O group.

The term "fluoro oil" is intended to mean an oil comprising at least one fluorine atom.

The term "hydrocarbon-based oil" is intended to mean an oil containing mainly hydrogen and carbon atoms.

The oils may optionally comprise oxygen, nitrogen, sulphur and/or phosphorus atoms, for example in the form of hydroxyl or acid radicals.

### Volatile oils

The volatile oils may be selected from hydrocarbon-based oils containing from 8 to 16 carbon atoms, and in particular branched C₈-C₁₆ alkanes (also known as isoparaffins), such as isododecane (also known as 2,2,4,4,6-pentamethylheptane), isodecane, or isohexadecane, and for example the oils sold under the trade names Isopars^{®} or Permethyls ^{®}.

Use may be made, as volatile oils, of volatile silicones, such as, for example, volatile linear or cyclic silicone oils, in particular those having a viscosity ≤ 8 centistokes (cSt) (8 × 10⁻⁶ m²/s) and having in particular from 2 to 10 silicon atoms, and in particular from 2 to 7 silicon atoms, these silicones optionally comprising alkyl or alkoxy groups having from 1 to 10 carbon atoms. As volatile silicone oil that can be used in the invention, mention may in particular be made of dimethicones having a viscosity of 5 and 6 cSt, octamethylcyclotetrasiloxane, decamethylcyclopentasiloxane, dodecamethyl-cyclohexasiloxane, heptamethylhexyltrisiloxane, heptamethyloctyltrisiloxane, hexamethyldisiloxane, octamethyltrisiloxane, decamethyltetrasiloxane, dodecamethyl-pentasiloxane, and mixtures thereof.

Use may also be made of volatile fluoro oils, such as nonafluoromethoxybutane or perfluoromethylcyclopentane, and mixtures thereof.

### Non-volatile oils

The non-volatile oils may in particular be selected from non-volatile hydrocarbon-based, fluoro and/or silicone oils.

As non-volatile hydrocarbon-based oil, mention may in particular be made of:
- hydrocarbon-based oils of animal origin,
- hydrocarbon-based oils of plant origin, such as phytostearyl esters, for example phytostearyl oleate, physostearyl isostearate and lauroyl/octyldodecyl/phytostearyl glutamate (Ajinomoto, Eldew PS203), triglycerides constituted of fatty acid esters of glycerol, in particular in which the fatty acids may have chain lengths ranging from C₄ to C₃₆, and in particular from C₁₈ to C₃₆, it being possible for these oils to be linear or branched, and saturated or unsaturated; these oils may in particular be heptanoic or octanoic triglycerides, shea oil, alfalfa oil, poppyseed oil, millet oil, barley oil, rye oil, candlenut oil, passion flower oil, shea butter, aloe oil, sweet almond oil, peach kernel oil, groundnut oil, argan oil, avocado oil, baobab oil, bourrache oil, broccoli oil, calendula oil, camelina oil, canola oil, carrot oil, safflower oil, hemp oil, rapeseed oil, cottonseed oil, coconut oil, marrow seed oil, wheat germ oil, jojoba oil, lily oil, macadamia oil, maize oil, meadowfoam oil, St. John's wort oil, monoi oil, hazelnut oil, apricot kernel oil, nut oil, olive oil, evening primrose oil, palm oil, blackcurrant seed oil, kiwi seed oil, grapeseed oil, pistachio oil, pumpkin oil, winter squash oil, quinoa oil, musk rose oil, sesame oil, soya oil, sunflower oil, castor oil and watermelon oil, and mixtures thereof, or alternatively caprylic/capric acid triglycerides, for instance those sold by the company Stéarineries Dubois or those sold under the name Miglyol 810^{®}, 812^{®} and 818^{®} by the company Dynamit Nobel,

- synthetic ethers having from 10 to 40 carbon atoms, such as dicapryl ether,
- synthetic esters, for instance oils of formula R1COOR2, in which R1 represents a linear or branched fatty acid residue containing from 1 to 40 carbon atoms and R2 represents a hydrocarbon-based chain, in particular a branched chain, containing from 1 to 40 carbon atoms, provided that R1 + R2 is ≥ 10. The esters may in particular be selected from fatty acid and alcohol esters, such as, for example, cetostearyl octanoate, isopropyl alcohol esters, such as isopropyl myristate, isopropyl palmitate, ethyl palmitate, 2-ethylhexyl palmitate, isopropyl stearate, octyl stearate, hydroxylated esters, for instance isostearyl lactate, octyl hydroxystearate, alcohol or polyalcohol ricinoleates, hexyl laurate, neopentanoic acid esters for instance isodecyl neopentanoate, isotridecyl neopentanoate, isononanoic acid esters, for instance isononyl isononanoate and isotridecyl isononanoate,
- polyol esters and pentaerythritol esters, for instance dipentaerythrityl tetrahydroxystearate/tetraisostearate,
- fatty alcohols that are liquid at ambient temperature, with a branched and/or unsaturated carbon chain containing from 12 to 26 carbon atoms, for instance 2-octyldodecanol, isostearyl alcohol and oleyl alcohol,
- C12-C22 higher fatty acids, such as oleic acid, linoleic acid, linolenic acid, and mixtures thereof,
- dialkyl carbonates, the 2 alkyl chains possibly being identical or different, such as the dicaprylyl carbonate sold under the name Cetiol CC® by Cognis, and
- oils of high molar mass having in particular a molar mass ranging from approximately 400 to approximately 2000 g/mol, in particular from approximately 650 to approximately 1600 g/mol. As oil of high molar mass that can be used in the present invention, mention may in particular be made of esters of linear fatty acids having a total carbon number ranging from 35 to 70, such as pentaerythrityl tetrapelargonate, hydroxylated esters, such as polyglycerol-2 triisostearate, aromatic esters, such as tridecyl trimellitate, esters of C24-C28 branched fatty acid or fatty alcohols, such as those described in Patent US 6,491,927, and pentaerythritol esters, and especially triisoarachidyl citrate, glyceryl triisostearate, glyceryl 2-tridecyl tetradecanoate, polyglyceryl-2

tetraisostearate or else pentaerythrityl 2-tetradecyl tetradecanoate; phenyl silicones, for instance Belsil PDM 1000 from the company Wacker (MM=9000 g/mol), non-volatile polydimethylsiloxanes (PDMSs), PDMSs comprising alkyl or alkoxy groups that are pendent and/or at the end of a silicone chain, these groups each containing from 2 to 24 carbon atoms, phenyl silicones, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, and dimethicones or phenyl trimethicone with a viscosity of less than or equal to 100 cSt, and mixtures thereof; and also the mixtures of these various oils.

According to one embodiment, a composition of the invention may advantageously comprise less than 10% by weight, or even less than 5% by weight, or even less than 2% by weight, relative to the total weight of the composition, or may even be devoid of silicone oil, in particular of cyclic silicone oil, and/or of mineral oil, and/or of branched volatile alkanes which are not directly derived from plants or of plant origin, such as isododecane or isoparaffins.

### Lipophilic structuring agent

A composition according to the invention may comprise at least one agent for structuring a liquid fatty phase, selected from a wax, a pasty compound, and mixtures thereof.

Such agents are used by those skilled in the art in contents that do not substantially affect the properties desired for the composition of the invention.

### Wax(es)

A composition of the invention may comprise at least one wax.

A wax that can be used in a composition of the invention may be selected from waxes that are solid at ambient temperature.

In particular, a wax suitable for the invention may be selected from waxes of animal, plant, mineral or synthetic origin, and mixtures thereof.

According to one embodiment variant, waxes of plant origin are preferred.

By way of illustration of waxes suitable for the invention, mention may in particular be made of hydrocarbon-based waxes, such as beeswax, lanoline wax, china insect waxes; rice brand wax, carnauba wax, candelilla wax, ouricury wax, asparta wax, berry wax, shellac wax, japan wax and sumac wax, montan wax, orange and lemon waxes, microcrystalline waxes, paraffins and ozokerite; polyethylene waxes, waxes obtained by Fisher-Tropsch synthesis and copolymer waxes, and esters thereof.

Mention may also be made of C₂₀-C₆₀ microcrystalline waxes, such as Microwax HW.

Mention may also be made of the MW 500 polyethylene wax sold under the reference Permalen 50-L polyethylene.

Mention may also be made of waxes obtained by catalytic hydrogenation of animal or plant waxes having linear or branched C₈-C₃₂ fatty chains.

Among these, mention may in particular be made of isomerized jojoba oil, such as the transisomerized partially hydrogenated jojoba oil manufactured or sold by the company Desert Whale under the commercial reference Iso-Jojoba-50^{®}, hydrogenated sunflower oil, hydrogenated castor oil, hydrogenated coconut oil, hydrogenated lanoline oil and di(1,1,1-trimethylolpropane) tetrastearate sold under the name Hest 2T-4S^{®} by the company Heterene.

Mention may also be made of silicone waxes (C₃₀-₄₅ Alkyl Dimethicone) and fluorinated waxes.

Use may also be made of the waxes obtained by hydrogenation of castor oil esterified with cetyl alcohol, which are sold under the names Phytowax ricin 16L64^{®} and 22L73^{®} by the company Sophim. Such waxes are described in application FR-A-2792190.

As wax, use may be made of a C₂₀-C₄₀ alkyl (hydroxystearyloxy) stearate (the alkyl group containing from 20 to 40 carbon atoms), alone or as a mixture.

Such a wax is in particular sold under the names Kester Wax K 82 P^{®}, Hydroxypolyester K 82 P^{®} and Kester Wax K 80 P^{®} by the company Koster Keunen.

According to one embodiment, a composition of the invention may advantageously comprise less than 10% by weight of silicone waxes and/or of mineral waxes, or even less than 5% by weight, or even less than 2% by weight of silicone waxes and/or of mineral waxes relative to the total weight of the composition, or may even be devoid of silicone waxes and/or mineral waxes.

### Pasty compounds

A composition according to the invention may comprise at least one pasty compound. The presence of a pasty compound may make it possible to advantageously confer improved comfort when a composition of the invention is deposited on keratin materials.

Such a compound may advantageously be selected from:
- lanoline and derivatives thereof,
- polymeric or nonpolymeric silicone compounds,
- polymeric or nonpolymeric fluorinated compounds,
- vinyl polymers, in particular:
   - olefin homopolymers,
   - olefin copolymers,
   - hydrogenated diene homopolymers and copolymers,
   - linear or branched and homo- or copolymeric oligomers of alkyl (meth)acrylates preferably having a C₈-C₃₀ alkyl group,
   - homo- and copolymeric oligomers of vinyl esters having C₈-C₃₀ alkyl groups,
   - homo- and copolymeric oligomers of vinyl ethers having C₈-C₃₀ alkyl groups,
- liposoluble polyethers resulting from polyetherification between one or more C₂-C₁₀₀, in particular C₂-C₅₀, diols,
- fatty acid or alcohol esters,
- and mixtures thereof.

Among the esters, mention may in particular be made of:
- the esters of an oligomeric glycerol, especially the esters of diglycerol, in particular the condensates of adipic acid and of glycerol, for which a portion of the hydroxyl groups of the glycerols have reacted with a mixture of fatty acids, such as steric acid, capric acid, stearic and isostearic acid and 12-hydroxystearic acid, such as, in particular, those sold under the trade mark Softisan 649 by the company Sasol, or such as bis-diglyceryl polyacyladipate-2,
- the arachidyl propionate sold under the trade mark Waxenol 801 by Alzo,
- phytosterol esters,
- triglycerides of fatty acids and derivatives thereof, such as hydrogenated cocoglycerides,
- noncrosslinked polyesters resulting from the polycondensation between a linear or branched C₄-C₅₀ dicarboxylic acid or polycarboxylic acid and a C₂-C₅₀ diol or polyol,
- aliphatic esters of an ester resulting from the esterification of an aliphatic hydroxycarboxylic acid ester with an aliphatic carboxylic acid,
- and mixtures thereof.

According to one embodiment, a composition of the invention may advantageously comprise less than 10% by weight of silicone and/or fluorinated pasty compounds, or even less than 5% by weight, or even less than 2% by weight of silicone and/or fluorinated pasty compounds, relative to the total weight of the composition, or may even be devoid of silicone and/or fluorinated pasty compounds.

### GELLING AGENTS

Depending on the fluidity of the composition that it is desired to obtain, one or more gelling agents can be incorporated into a composition of the invention.

A gelling agent suitable for the invention may be hydrophilic, i.e. soluble or dispersible in water.

Said gelling agents may in particular be selected from: modified or nonmodified carboxyvinyl polymers, such as the Carbopols (CTFA name: carbomer) sold by the company Goodrich; polyacrylates and polymethacrylates, such as the products sold under the names Lubrajel and Norgel by the company Guardian; polyacrylamides; polymers and copolymers of 2-acrylamido-2-methylpropanesulphonic acid, which are optionally crosslinked and/or neutralized, such as the poly(2-acrylamido-2-methylpropanesulphonic acid) sold by the company Clariant under the name Hostacerin AMPS (CTFA name: ammonium polyacryldimethyltauramide); crosslinked anionic acrylamide/AMPS copolymers, in the form of a W/O emulsion, such as those sold under the name Sepigel 305 (CTFA name: Polyacrylamide/C₁₃₋₁₄ Isoparaffin/Laureth-7) and under the name Simulgel 600 (CTFA name: Acrylamide/Sodium acryloyldimethyltaurate copolymer/Isohexadecane/Polysorbate 80) by the company SEPPIC; polysaccharide biopolymers, such as xanthan gum, guar gum, carob gum, acacia gum, scleroglucans, chitin derivatives and chitosan derivatives, carrageenans, gellans, alginates or celluloses such as microcrystalline cellulose, carboxymethylcellulose, hydroxymethylcellullose and hydroxypropylcellulose; and mixtures thereof.

A gelling agent suitable for the invention may be lipophilic. A lipophilic gelling agent may be inorganic or organic.

As lipophilic gelling agents, mention may, for example, be made of modified clays, such as modified magnesium silicate (Bentone gel VS38 from Rheox), hectorite modified with distearyldimethylammonium chloride (CTFA name: Disteardimonium hectorite) sold under the name Bentone 38 CE by the company Rheox.

As inorganic lipophilic gelling agent, mention may be made of optionally modified clays, such as hectorites modified with a C₁₀ to C₂₂ fatty acid ammonium chloride, for instance hectorite modified with distearyldimethylammonium chloride, such as, for example, that sold under the name Bentone 38V^{®} by the company Elementis.

The polymeric organic lipophilic gelling agents are, for example, partially or totally crosslinked elastomeric organopolysiloxanes with a three-dimensional structure, such as those sold under the names KSG6^{®}, KSG16^{®} and KSG18^{®} by the company Shin-Etsu, Trefil E-505C^{®} and Trefil E-506C^{®} by the company Dow-Corning, Gransil SR-CYC^{®}, SR DMF10^{®}, SR-DC556^{®}, SR 5CYC gel^{®}, SR DMF 10 gel^{®} and SR DC 556 gel^{®} by the company Grant Industries, and SF 1204^{®} and JK 113^{®} by the company General Electric; block copolymers of "diblock", "triblock" or "radial" type, of the polystyrene/polyisoprene or polystyrene/polybutadiene type, such as those sold under the name Luvitol HSB^{®} by the company BASF, of the polystyrene/copoly(ethylenepropylene) type, such as those sold under the name Kraton^{®} by the company Shell Chemical Co, or else of the polystyrene/copoly(ethylene-butylene) type, blends of triblock and radical (star) copolymers in isododecane, such as those sold by the company PENRECO under the name Versagel^{®}, for instance the mixture of butylene/ethylene/styrene triblock copolymer and of ethylene/propylene/styrene star copolymer in isododecane (Versagel M 5960).

Among the lipophilic gelling agents that may be used in a cosmetic composition of the invention, mention may also be made of esters of dextrin and of a fatty acid, such as dextrin palmitates, in particular such as those sold under the name Rheopearl TL^{®}, Rheopearl TL2-OR^{®} or Rheopearl KL^{®} by the company Chiba Flour.

By way of lipophilic gelling agents suitable for the invention, mention may also be made of hydrogenated plant oils, such as hydrogenated castor oil.

By way of lipophilic gelling agent also suitable for the invention, mention may be made of fatty alcohols, in particular C₈ to C₂₆ fatty alcohols, and more particularly C₁₂ to C₂₂ fatty alcohols.

According to one embodiment, a fatty alcohol suitable for the invention may be selected from mysrityl alcohol, cetyl alcohol, stearyl alcohol and behenyl alcohol.

By way of lipophilic gelling agent also suitable for the invention, mention may be made of fatty acid esters of glycerols, such as glyceryl stearate.

According to one embodiment, a composition of the invention may comprise at least one lipophilic gelling agent, in particular selected from modified hectorites.

According to one embodiment, a composition of the invention may advantageously comprise less than 10% by weight of silicone gelling agents, or even less than 5%, or even less than 2% by weight of silicone gelling agents, relative to the total weight of the composition, or may even be devoid of silicone gelling agents.

### COLORANTS

A composition according to the invention may also comprise at least one colorant.

A cosmetic composition in accordance with the invention may advantageously incorporate at least one colorant selected from organic or inorganic colorants, in particular such as pigments or nacres conventionally used in cosmetic compositions, which may be fat-soluble or water-soluble, materials with a specific optical effect, and mixtures thereof.

The term "pigments" should be understood to mean white or coloured, inorganic or organic particles which are insoluble in an aqueous solution and are intended for colouring and/or opacifying the resulting film.

The pigments may be present in a proportion of from 0.01% to 40% by weight, especially from 0.1 % to 20% by weight, and in particular from 1% to 15% by weight, relative to the total weight of the cosmetic composition.

As inorganic pigments that can be used in the invention, mention may be made of titanium dioxide, zirconium oxide or cerium oxide, and also zinc oxide, iron oxide or chromium oxide, ferric blue, manganese violet, ultramarine blue and chromium hydrate.

The pigment may also be a pigment having a structure which may be, for example, of sericite/brown iron oxide/titanium dioxide/silica type. Such a pigment is sold, for example, under the reference Coverleaf NS or JS by the company Chemicals and Catalysts and has a contrast ratio of around 30.

The colorant may also comprise a pigment having a structure which may be, for example, of the type of silica microspheres containing iron oxide. An example of a pigment having this structure is sold by the company Miyoshi under the reference PC BALL PC-LL-100 P, this pigment being composed of silica microspheres containing yellow iron oxide.

Among the organic pigments that can be used in the invention, mention may be made of carbon black, D & C pigments, lakes based on cochineal carmine, on barium, strontium, calcium or aluminium, or else the diketopyrrolopyrroles (DPPs) described in documents EP-A-542669, EP-A-787730, EP-A-787731 and WO-A-96/08537.

The term "nacres" should be understood to mean iridescent or non-iridescent coloured particles of any shape, which are in particular produced by certain molluscs in their shell or else are synthesized, and which exhibit a colour effect by optical interference.

The nacres may be selected from pearlescent pigments such as titanium mica coated with an iron oxide, titanium mica coated with bismuth oxychloride, titanium mica coated with chromium oxide, titanium mica coated with an organic dye, and pearlescent pigments based on bismuth oxychloride. This may also involve mica particles at the surface of which are superposed at least two successive layers of metal oxides and/or of organic colorants.

By way of example of nacres, mention may also be made of natural mica coated with titanium dioxide, with iron oxide, with natural pigment or with bismuth oxychloride.

Among the nacres available on the market, mention may be made of the nacres Timica, Flamenco and Duochrome (based on mica), sold by the company Engelhard, the Timiron nacres sold by the company Merck, the Prestige mica-based nacres sold by the company Eckart and the synthetic-mica-based Sunshine nacres sold by the company Sun Chemical.

The nacres may more particularly possess a yellow, pink, red, bronze, orange, brown, gold and/or copper colour or glint.

By way of illustration of nacres which can be used in the context of the present invention, mention may be made in particular of the golden nacres sold in particular by the company Engelhard, under the names Brilliant gold 212G (Timica), Gold 222C (Cloisonne), Sparkle gold (Timica), Gold 4504 (Chromalite) and Monarch gold 233X (Cloisonne); the bronze nacres sold in particular by the company Merck under the name Bronze fine (17384) (Colorona) and Bronze (17353) (Colorona) and by the company Engelhard under the name Super bronze (Cloisonne); the orange nacres sold in particular by the company Engelhard under the name Orange 363C (Cloisonne) and Orange MCR 101 (Cosmica) and by the company Merck under the name Passion orange (Colorona) and Matte orange (17449) (Microna); the brown-hued nacres sold in particular by the company Engelhard under the name Nu-antique copper 340XB (Cloisonne) and Brown CL4509 (Chromalite); the copper-glint nacres sold in particular by the company Engelhard under the name Copper 340A (Timica); the red-glint nacres sold in particular by the company Merck under the name Sienna fine (17386) (Colorona); the yellow-glint nacres sold in particular by the company Engelhard under the name Yellow (4502) (Chromalite); the gold-glint red-hued nacres sold in particular by the company Engelhard under the name Sunstone G012 (Gemtone); the pink nacres sold in particular by the company Engelhard under the name Tan opale G005 (Gemtone); the gold-glint black nacres sold in particular by the company Engelhard under the name Nu antique bronze 240 AB (Timica), the blue nacres sold in particular by the company Merck under the name Matte blue (17433) (Microna), the silver-glint white nacres sold in particular by the company Merck under the name Xirona Silver and the green-golden pinkish orangey nacres sold in particular by the company Merck under the name Indian summer (Xirona), and mixtures thereof.

The cosmetic composition according to the invention may also comprise water-soluble or fat-soluble dyes. The fat-soluble dyes are, for example, Sudan red, DC Red 17, DC Green 6, β-carotene, Sudan brown, DC Yellow 11, DC Violet 2, DC orange 5 and quinoline yellow. The water-soluble dyes are, for example, beetroot juice and caramel.

The cosmetic composition according to the invention may also contain at least one material with a specific optical effect.

This effect is different from a simple, conventional hue effect, i.e. a unified and stabilized effect of the colour as produced by conventional colorants, such as, for example, monochromic pigments. For the purpose of the invention, the term "stabilized" signifies absence of an effect of variability of colour with the angle of observation or else in response to a temperature change.

For example, this material may be selected from particles having a metallic glint, goniochromatic colouring agents, diffracting pigments, thermochromic agents, optical brighteners, and also fibres, in particular of interference type. Of course, these various materials may be combined so as to provide the simultaneous manifestation of two effects, or even of a new effect in accordance with the invention.

The metallic-glint particles that can be used in the invention are in particular selected from:
- particles of at least one metal and/or at least one metal derivative,
- particles comprising a single-substance or multi-substance, organic or inorganic substrate, at least partially coated with at least one metal-glint layer comprising at least one metal and/or at least one metal derivative, and
- mixtures of said particles.

Among the metals that may be present in said particles, mention may, for example, be made of Ag, Au, Cu, Al, Ni, Sn, Mg, Cr, Mo, Ti, Zr, Pt, Va, Rb, W, Zn, Ge, Te, Se and mixtures or alloys thereof. Ag, Au, Cu, Al, Zn, Ni, Mo, Cr and mixtures or alloys thereof (for example, bronzes and brasses) are preferred metals.

The term "metal derivatives" denotes compounds derived from metals, in particular oxides, fluorides, chlorides and sulphides.

By way of illustration of these particles, mention may be made of aluminium particles, such as those sold under the names Starbrite 1200 EAC^{®} by the company Siberline and Metalure^{®} by the company Eckart.

Mention may also be made of metal powders of copper or of alloy mixtures, such as the references 2844 sold by the company Radium Bronze, metal pigments, such as aluminium or bronze, for instance those sold under the name Rotosafe 700 by the company Eckart, the silica-coated aluminium particles sold under the name Visionaire Bright Silver by the company Eckart, and the metal alloy particles such as silica-coated bronze (copper and zinc alloy) powders sold under the name Visionaire Bright Natural Gold by the company Eckart.

The particles in question may also be particles comprising a glass substrate, such as those sold by the company Nippon Sheet Glass under the name Microglass Metashine.

The goniochromatic colouring agent may be selected, for example, from multilayer interference structures and liquid-crystal colouring agents.

Examples of symmetrical multilayer interference structures that can be used in compositions prepared in accordance with the invention are, for example, the following structures: Al/SiO₂/Al/SiO₂/Al, pigments having this structure being sold by the company Dupont De Nemours; Cr/MgF₂/Al/MgF₂/Cr, pigments having this structure being sold under the name Chromaflair by the company Flex; MoS₂/SiO₂/Al/SiO₂/MoS₂; Fe₂O₃/SiO₂/Al/SiO₂/Fe₂O₃, and Fe₂O₃/SiO₂/Fe₂O₃/SiO₂/Fe₂O₃, pigments having these structures being sold under the name Sicopearl by the company BASF; MoS₂/SiO₂/mica-oxide/SiO₂/MoS₂; Fe₂O₃/SiO₂/mica-oxide/SiO₂/Fe₂O₃; TiO₂/SiO₂/TiO₂ and TiO₂/Al₂O₃/TiO₂; SnO/TiO₂/SiO₂/TiO₂/SnO; Fe₂O₃/SiO₂/Fe₂O₃; SnO/mica/TiO₂/SiO₂/TiO₂/mica/SnO, pigments having these structures being sold under the name Xirona by the company Merck (Darmstadt). By way of example, these pigments may be the pigments of silica/titanium oxide/tin oxide structure sold under the name Xirona Magic by the company Merck, the pigments of silica/brown iron oxide structure sold under the name Xirona Indian Summer by the company Merck, and the pigments of silica/titanium oxide/mica/tin oxide structure sold under the name Xirona Carribean Blue by the company Merck. Mention may also be made of the Infinite Colours pigments from the company Shiseido. Depending on the thickness and the nature of the various layers, different effects are obtained. Thus, with the Fe₂O₃/SiO₂/Al/SiO₂/Fe₂O₃ structure, the colour changes from green-golden to red-grey for SiO₂ layers of 320 to 350 nm; from red to golden for SiO₂ layers of 380 to 400 nm; from violet to green for SiO₂ layers of 410 to 420 nm; from copper to red for SiO₂ layers of 430 to 440 nm.

By way of example of pigments with a polymeric multilayer structure, mention may be made of those sold by the company 3M under the name Color Glitter.

Examples of liquid-crystal goniochromatic particles that may be used include those sold by the company Chenix, and also the product sold under the name Helicone HC by the company Wacker.

### FILLERS

A composition in accordance with the invention may also comprise at least one filler, of organic or inorganic nature.

The term "filler" should be understood to mean colourless or white solid particles of any shape, which are in a form that is insoluble and dispersed in the medium of the composition. Inorganic or organic in nature, they make it possible to confer softness, a matt aspect and uniformity of make-up on the composition.

The fillers used in the compositions according to the present invention may be of lamellar, globular or spherical form, in the form of fibres, or in any other form intermediate between these defined forms.

The fillers according to the invention may or may not be surface-coated, and in particular they may be surface-treated with silicones, amino acids, fluoro derivatives, or any other substance that promotes the dispersion and compatibility of the filler in the composition.

Among the inorganic fillers that can be used in the compositions according to the invention, mention may be made of talc, mica, silica, trimethyl siloxysilicate, kaolin, bentone, calcium carbonate and magnesium hydrogen carbonate, hydroxyapatite, boron nitride, hollow silica microspheres (silica beads from Maprecos), glass or ceramic microcapsules, silica-based fillers such as Aerosil 200, Aerosil 300; Sunsphere H-33, Sunsphere H-51 sold by Asahi Glass; Chemicelen sold by Asahi Chemical; composites of silica and of titanium dioxide, such as the TSG series sold by Nippon Sheet Glass, and mixtures thereof.

A filler suitable for the invention may preferably be calcium carbonate.

Among the organic fillers that can be used in the compositions according to the invention, mention may be made of polyamide powders (Nylon^{®} Orgasol from Atochem), poly-β-alanine powders and polyethylene powders, polytetrafluoroethylene powders (Teflon^{®}), lauroylysine, starch, tetrafluoroethylene polymer powders, hollow polymer microspheres such as Expancel (Nobel Industrie), metal soaps derived from organic carboxylic acids containing from 8 to 22 carbon atoms, preferably from 12 to 18 carbon atoms, for example zinc stearate, magnesium stearate or lithium stearate, zinc laurate, magnesium myristate, Polypore^{®} L 200 (Chemdal Corporation), silicone resin microbeads (for example, Tospearl^{®} from Toshiba), polyurethane powders, in particular powders of crosslinked polyurethane comprising a copolymer, said copolymer comprising trimethylol hexyllactone, for instance the hexamethylene diisocyanate/trimethylol hexyllactone polymer sold under the name Plastic Powder D-400^{®} or Plastic Powder D-800^{®} by the company Toshiki, carnauba microwaxes, such as the product sold under the name MicroCare 350^{®} by the company Micro Powders, synthetic-wax microwaxes, such as the product sold under the name MicroEase 114S^{®} by the company Micro Powders, microwaxes constituted of a mixture of carnauba wax and of polyethylene wax, such as those sold under the names Micro Care 300^{®} and 310^{®} by the company Micro Powders, microwaxes constituted of a mixture of carnauba wax and of synthetic wax, such as the product sold under the name Micro Care 325^{®} by the company Micro Powders, polyethylene microwaxes, such as those sold under the names Micropoly 200^{®}, 220^{®}, 220L^{®} and 250S^{®} by the company Micro Powders; and mixtures thereof.

A filler suitable for the invention may preferably be selected from natural microwaxes or microwaxes of natural origin, such as carnauba microwaxes.

### ACTIVE AGENTS

A composition of the invention may also comprise at least one cosmetic active agent and/or one dermatological active agent.

By way of non-limiting examples of cosmetic and/or dermatological active agents suitable for the invention, mention may be made of the active agents selected from the following agents:
moisturizers, desquamating agents, barrier function enhancers, depigmenting agents, antioxidants, dermodecontracting agents, anti-glycation agents, agents for stimulating the synthesis of dermal and/or epidermal macromolecules and/or preventing their degradation, agents for stimulating fibroblast or keratinocyte proliferation and/or keratinocyte differentiation, agents for promoting maturation of the horny envelope, NO-synthase inhibitors, peripheral benzodiazepine receptor (PBR) antagonists, agents for increasing sebaceous gland activity, agents for stimulating the energy metabolism of cells, tensioning agents, fat-restructuring agents, slimming agents, agents for promoting cutaneous microcirculation, calmatives and/or anti-irritants, sebum-regulating or anti-seborrheic agents, astringents, cicatrising agents, anti-inflammatories, anti-acne agents, matting agents, soft-focus effect fillers, fluorescent agents, agents for promoting the natural pinkish colouring of the skin, abrasive or exfoliant fillers, antimicrobial agents, and calmatives, anti-NO agents, free-radical scavengers or anti-pollution agents, sunscreens, vitamins such as tocopherol, and mixtures thereof.

The cosmetic and/or dermatological active agents suitable for the invention may in particular be selected from the agents mentioned in application EP 1 847 247.

According to one embodiment, a composition of the invention for use in protecting keratin materials, and in particular the skin of the body or the lips, against sunlight may in particular comprise, as sunscreens, cinnamic derivatives, salicylic derivatives, camphor derivatives, triazine derivatives, benzophenone derivatives, dibenzolemethane derivatives, β-diphenylacrylate derivatives, p-aminobenzoic acid derivatives, screening polymers and screening silicones, as described, for example, in application WO 93/04665.

In addition, a composition of the invention may contain agents for artificially tanning and/or browning the skin, for instance dihydroxyacetone (DHA).

It is part of the abilities of those skilled in the art to select said agent(s) and the content thereof as a function of the desired effect on the keratin materials, and so as not to affect the cosmetic properties and the properties of comfort on application and of stability, of the compositions of the invention.

### ADDITIVES

A cosmetic composition according to the invention may also further comprise any additive normally used in the field under consideration, for example selected from film-formers and, where appropriate, film-forming auxiliaries, gums, semi-crystalline polymers, antioxidants, essential oils, preservatives, fragrances, neutralizing agents, antiseptic agents, anti-UV protective agents, and mixtures thereof.

Those skilled in the art can adjust the nature and amount of the additives present in the compositions in accordance with the invention by means of routine operations, such that the cosmetic properties and the properties of comfort on application and of stability that are desired for these compositions are not thereby affected.

A composition according to the invention may in particular be in the form of a make-up and/or care composition for the skin or the lips, in particular a lipstick or a lip balm.

According to one embodiment, a composition of the invention may advantageously be in the form of a foundation.

A composition of the invention may be obtained by means of any method of preparation known to those skilled in the art.

The present invention will be understood more clearly by means of the following examples.

These examples are provided only by way of illustration of the invention and should not be interpreted as limiting the scope thereof.

### EXAMPLES

### Examples 1 to 3:

The following three foundation examples show the influence of the polyglycerol polyricinoleates of the invention on the stability of the composition.

| **Compounds/trade names** | | **Ex. 1 (Invention)** | **Ex. 2 (Invention)** | **Ex.3 (Comparative)** |
|---|---|---|---|---|
| **A1** | Dicapryl ether sold under the name Cetiol OE by the company Cognis | 10.0 | 10.0 | 10.0 |
| **A2** | Polyglyceryl-3 polyricinoleate sold under the name Akoline PGPR by the company Karlshamns | **6.3** | **0** | **0** |
| | Polyglyceryl-6 polyricinoleate sold under the name SFace CR-1001 by the company Sakamoto Yakuhin Kogyo Co. Ltd | **0** | **6.3** | **0** |
| | Polyglyceryl-2 dipolyhydroxysterate sold under the name Dehymuls PGPH by the company Cognis | **0** | **0** | **6.3** |
| **A3** | Macadamia oil | 5.1 | 5.1 | 5.1 |
| **A4** | Undecane+Tridecane sold under the name Cetiol UT by the company Cognis | 16.3 | 16.3 | 16.3 |
| **B** | Demineralized water | 44.6 | 44.6 | 44.6 |
| | Glycerol | 6.3 | 6.3 | 6.3 |
| | Sodium chloride | 1.3 | 1.3 | 1.3 |
| | Sorbitol in an aqueous solution at 70%, sold under the name Neosorb 70/70 B by the company Roquette | 2.5 | 2.5 | 2.5 |
| | Benzyl alcohol | 1.3 | 1.3 | 1.3 |
| | Ethanol | 6.3 | 6.3 | 6.3 |
| | TOTAL | 100% by mass | 100% by mass | 100% by mass |

The ingredient of phase A1 is weighed into a main beaker, and then placed on a magnetic stirring plate, with stirring (magnetic bar, 200 rpm) and heated to 60°C.

Phase A2 is added, with stirring and heating maintained until a homogeneous mixture is obtained. The mixture is left to cool to ambient temperature with stirring. Phases A3 and then A4 are subsequently added.

Phase B, the aqueous phase, is prepared by adding the water heated to 95°C to the mixture comprising the glycerol, the sodium chloride and the sorbitol previously weighed out. The benzyl alcohol and the ethanol are then introduced at a temperature below 40°C.

Emulsification takes place at ambient temperature: the aqueous phase is poured into the fatty phase, while the stirring speed is gradually increased to 4500 rpm.

Stirring is maintained for 10 minutes.

### Stability measurement

The stability of the emulsion is measured using particle-size measurements, as indicated previously.

More specifically, 25 ml of each of the foundation formulations are stored at 45°C.

The particle-size measurements are carried out on the actual day of production, and also after 14 and 30 days of storage.

These measurements are carried out using a particle sizer (Mastersizer 2000 from Malvern Instruments coupled to central function control software) which makes it possible to determine the particle size distribution by light diffraction, according to the supplier's recommendations.

The sample feed system is a Hydro 2000 SM "small-volume manual sample presenting system".

The measurements are carried out in an anhydrous phase in isohexadecane. The sample is added dropwise to the "sample presenting system" until between 10% and 20% obscuration is obtained, and then the measurement is carried out.

The result obtained is the value of the volume mean diameter D [4,3], expressed in µm.

### Results

The stability results corresponding, respectively, to the formulations of Examples 1, 2 and 3 are reported in the following table:

| **Storage time at 45°C, days** | **Volume mean diameter of the drops D [4,3], µm** | | |
|---|---|---|---|
| | Ex. 1 | Ex. 2 | Ex. 3 |
| 0 | 1.11 | 0.92 | 0.92 |
| 14 | 2.26 | 1.11 | 2.25 |
| 30 | 2.73 | 1.74 | 3.13 |

At 30 days, the emulsion of Example 3 (comparative example) has a larger globule size than that of the emulsions according to the invention (Examples 1 and 2).

In addition, after two months of storage at 45°C, the emulsion of Example 3 (comparative example) visually displays much more sedimentation than that observed for the other two emulsions of the compositions of the invention.

The results thus reveal that the emulsions according to the invention show improved stability compared with the other emulsions.

### Examples 4 and 5:

The foundation examples which follow make it possible to compare the influence of the volatile linear alkanes on the stability of the composition.

| **Compounds/trade names** | | **Ex.4 (Invention)** | **Ex.5 (Comparative)** |
|---|---|---|---|
| **A1** | Dicapryl ether sold under the name Cetiol OE by the company Cognis | 3.90 | 3.90 |
| | Modified hectorite sold under the name Bentone 38 VCG by the company Elementis | 1.20 | 1.20 |
| **A2** | Undecane+Tridecane sold under the name Cetiol UT by the company Cognis | **16.10** | **0** |
| | Dicapryl ether sold under the name Cetiol OE by the company Cognis | **0** | **16.10** |
| **A3** | Polyglyceryl-3 polyricinoleate sold under the name Akoline PGPR by the company Karlshamns | 4.75 | 4.75 |
| **A4** | Dicapryl ether sold under the name Cetiol OE by the company Cognis | 5.00 | 5.00 |
| | Yellow iron oxide coated with aluminium stearoylglutamate | 2.52 | 2.52 |
| | Red iron oxide coated with aluminium stearoylglutamate | 0.69 | 0.69 |
| | Black iron oxide coated with aluminium stearoylglutamate | 0.29 | 0.29 |
| | Titanium dioxide coated with aluminium stearoylglutamate | 10.50 | 10.50 |
| **A5** | Mixture of natural tocopherols with soya oil (50/50) | 0.50 | 0.50 |
| **A6** | Natural calcium carbonate sold under the name Omyapure 35 LM-OG by the company Omya | 5.00 | 5.00 |
| **B** | Demineralized water | 33.15 | 33.15 |
| | Glycerol | 5.00 | 5.00 |
| | Sodium chloride | 0.60 | 0.60 |
| | Sorbitol in an aqueous solution at 70%, sold under the name Neosorb 70/70 B by the company Roquette | 5.00 | 5.00 |
| | Benzyl alcohol | 0.80 | 0.80 |
| **C** | Ethanol | 5.00 | 5.00 |
| | TOTAL | 100% by mass | 100% by mass |

The dicapryl ether of phase A1 is weighed out into a main beaker, and then placed on a magnetic stirring hotplate. The hectorite is then incorporated with stirring (magnetic bar, 200 rpm) and with heating (80°C).

The stirring is maintained until a homogeneous mixture is obtained. The mixture is then cooled to ambient temperature while maintaining the stirring, and then phases A2 and A3 are added.

Phase A4 is prepared separately by grinding the mixture (pigments/dicapryl ether) using a ball mill (Dynomill), and is then added to the main beaker, with stirring

(Moritz stirrer 1500 rpm).

Finally, phases A5 and then A6 are introduced, with stirring being maintained.

Phase B, the aqueous phase, is prepared by adding water heated to 95°C to the mixture comprising the glycerol, the sodium chloride and the sorbitol previously weighed out.

The benzyl alcohol is then introduced at a temperature below 40°C.

Emulsification takes place at ambient temperature: the aqueous phase is poured into the fatty phase, while the stirring speed is gradually increased to 4500 rpm.

Stirring is maintained for 10 minutes.

Finally, phase C is added, with stirring being maintained until complete incorporation thereof.

### Stability measurement

The stability of the composition is evaluated by observation of a sample of the composition under a microscope (magnification X100) after 48 hours at ambient temperature.

### Results:

The stability results corresponding, respectively, to Examples 4 and 5 are reported in the following table:

| | **Ex 4 (Invention)** | **Ex 5 (Comparative)** |
|---|---|---|
| **Oil** | Undecane+Tridecane sold under the name Cetiol UT by the company Cognis | Dicapryl ether sold under the name Cetiol OE by the company Cognis |
| **Stability, microscopic appearance** | Emulsion stable after 48 h. A few scattered drops. | Emulsion unstable after 48 h. Emulsion coarse with separations. |

The results indicate that the emulsion of the invention has an improved stability.

### Examples 6 - 7

The foundation examples which follow make it possible to compare the influence of the volatile linear alkanes (Example 6) relative to isododecane (Example 7) on the cosmetic properties of the composition.

| **Compounds/trade names** | | **Ex. 6 (Invention)** | **Ex. 7 (comparative)** |
|---|---|---|---|
| **A1** | Dicapryl ether sold by the name Cetiol OE by the company Cognis | 3.90 | 3.90 |
| | Modified hectorite sold under the name Bentone 38 VCG by the company Elementis | 1.20 | 1.20 |
| **A2** | Undecane+Tridecane sold under the name Cetiol UT by the company Cognis | **16.10** | **0** |
| | Isododecane | **0** | **16.10** |
| **A3** | Polyglyceryl-3 polyricinoleate sold under the name Akoline PGPR by the company Karlshamns | 4.75 | 4.75 |
| **A4** | Dicapryl ether sold under the name Cetiol OE by the company Cognis | 5.00 | 5.00 |
| | Yellow iron oxide coated with aluminium stearoylglutamate | 2.52 | 2.52 |
| | Red iron oxide coated with aluminium stearoylglutamate | 0.69 | 0.69 |
| | Black iron oxide coated with aluminium stearoylglutamate | 0.29 | 0.29 |
| | Titanium dioxide coated with aluminium stearoylglutamate | 10.50 | 10.50 |
| **A5** | Mixture of natural tocopherols with soya oil (50/50) | 0.50 | 0.50 |
| **A6** | Natural calcium carbonate sold under the name Omyapure 35 LM-OG by the company Omya | 5.00 | 5.00 |
| **B** | Demineralized water | 33.15 | 33.15 |
| | Glycerol | 5.00 | 5.00 |
| | Sodium chloride | 0.60 | 0.60 |
| | Sorbitol in an aqueous solution at 70%, sold under the name Neosorb 70/70 B by the company Roquette | 5.00 | 5.00 |
| | Benzyl alcohol | 0.80 | 0.80 |
| **C** | Ethanol | 5.00 | 5.00 |
| | TOTAL | 100% by mass | 100% by mass |

The compositions are prepared as indicated in Examples 4-5.

### Sensoral evaluation

The foundations obtained are evaluated by users with regard to the cosmetic aspects, the make-up result and the comfort.

The evaluation is carried out after free application, on a panel of 5 expert individuals, of an amount of 0.1 g of each of the compositions per half face.

### Results

From the results of the study, it is concluded that the cosmetic properties of the foundation of Example 6, according to the invention, are better than those observed for the foundation of Example 7, which is not part of the invention.

The foundation according to the invention, Example 6, dries less rapidly, and it gives a make-up result that is less tacky, slides better and is more comfortable.

### Example 8:

### Foundation-W/O emulsion

| **Compounds/trade names** | | **% by mass** |
|---|---|---|
| **A1** | Dicapryl ether sold under the name Cetiol OE by the company Cognis | 6.00 |
| | Modified hectorite sold under the name Bentone 38 VCG by the company Elementis | 1.20 |
| **A2** | Undecane+Tridecane sold under the name Cetiol UT by the company Cognis | 8.00 |
| **A3** | Polyglyceryl-3 polyricinoleate sold under the name Akoline PGPR by the company Karlshamns | 4.75 |
| **A4** | Dicapryl ether sold under the name Cetiol OE by the company Cognis | 5.00 |
| | Yellow iron oxide coated with aluminium stearoylglutamate | 2.52 |
| | Red iron oxide coated with aluminium stearoylglutamate | 0.69 |
| | Black iron oxide coated with aluminium stearoylglutamate | 0.29 |
| | Titanium dioxide coated with aluminium stearoylglutamate | 10.50 |
| **A5** | Macadamia oil | 6.00 |
| | Mixture of natural tocopherols with soya oil (50/50) | 0.50 |
| **A6** | Natural calcium carbonate sold under the name Omyapure 35 LM-OG by the company Omya | 5.00 |
| **B** | Demineralized water | 33.15 |
| | Glycerol | 5.00 |
| | Sodium chloride | 0.60 |
| | Sorbitol in an aqueous solution at 70%, sold under the name Neosorb 70/70 B by the company Roquette | 5.00 |
| | Benzyl alcohol | 0.80 |
| **C** | Ethanol | 5.00 |

The composition is prepared as indicated in Example 4.

### Result:

This foundation is stable and has good cosmetic properties.

### Example 9:

### Foundation-W/O emulsion

| **Compounds/trade names** | | **% by mass** |
|---|---|---|
| **A1** | Dicapryl ether sold under the name Cetiol OE by the company Cognis | 4.00 |
| | Modified hectorite sold under the name Bentone 38 VCG by the company Elementis | 1.20 |
| **A2** | Undecane+Tridecane sold under the name Cetiol UT by the company Cognis | 14.00 |
| **A3** | Polyglyceryl-6 polyricinoleate sold under the name SFace CR-1001 by the company Sakamoto Yakuhin Kogyo Co. Ltd | 6 |
| **A4** | Dicapryl ether sold under the name Cetiol OE by the company Cognis | 5 |
| | Yellow iron oxide coated with aluminium stearoylglutamate | 2.52 |
| | Red iron oxide coated with aluminium stearoylglutamate | 0.69 |
| | Black iron oxide coated with aluminium stearoylglutamate | 0.29 |
| | Titanium dioxide coated with aluminium stearoylglutamate | 10.5 |
| **A5** | Macadamia oil | 2 |
| | Mixture of natural tocopherols with soya oil (50/50) | 0.5 |
| **A6** | Natural calcium carbonate sold under the name Omyapure 35 LM-OG by the company Omya | 5 |
| **B** | Demineralized water | 29.3 |
| | Glycerol | 5 |
| | Sodium chloride | 0.2 |
| | Sorbitol in an aqueous solution at 70%, sold under the name Neosorb 70/70 B by the company Roquette | 8 |
| | Benzyl alcohol | 0.8 |
| **C** | Ethanol | 5 |
| | TOTAL | 100% |

The composition is prepared as indicated in Example 4.

### Result:

This foundation is stable and has good cosmetic properties.

## Claims

1. Emulsion-type cosmetic and/or dermatological composition comprising a fatty continuous phase, said composition comprising at least one polyglyceryl polyricinoleate, at least one polyol and at least one volatile linear alkane.

2. Composition according to the preceding claim, in which the composition is of water-in-oil emulsion type.

3. Composition according to Claim 1 or 2, in which said alkane contains from 7 to 17 carbon atoms, and more particularly from 9 to 15 carbon atoms, in particular is selected from n-nonane, n-decane, n-undecane, n-dodecane, n-tridecane, n-tetradecane, n-pentadecane, n-hexadecane and n-heptadecane, and mixtures thereof.

4. Composition according to any one of the preceding claims, in which said alkane is used in the form of an n-undecane/n-tridecane mixture.

5. Composition according to any one of the preceding claims, comprising from 1% to 50% by weight, in particular from 5% to 35% by weight, and more preferentially from 8% to 25% by weight of volatile linear alkane(s), relative to the total weight of the composition.

6. Composition according to any one of the preceding claims, in which the polyglyceryl polyricinoleate results from the esterification of at least one polyglycerol selected from the compounds of formula (I) below: 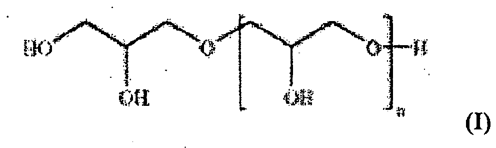 in which n denotes an integer between 1 and 11, and more particularly between 1 and 7; and at least one polyricinoleic acid selected from the compounds of formula (II) below: 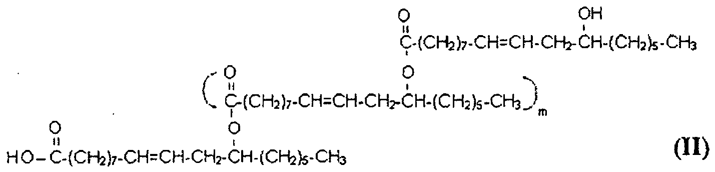 in which m denotes an integer between 0 and 10, in particular between 1 and 8, and more particularly between 1 and 5.

7. Composition according to any one of the preceding claims, in which said polyglyceryl polyricinoleate is selected from polyglyceryl-3 polyricinoleate, polyglyceryl-5 polyricinoleate, polyglyceryl-6 polyricinoleate and polyglyceryl-10 polyricinoleate, and more particularly is selected from polyglyceryl-3 polyricinoleate and polyglyceryl-6 polyricinoleate, and mixtures thereof.

8. Composition according to any one of the preceding claims, comprising from 2% to 10% by weight, in particular from 3% to 8% by weight, and more particularly from 4% to 7% by weight of polyglycerol polyricinoleate(s), relative to the total weight of the composition.

9. Composition according to any one of the preceding claims, in which said polyol is selected from saturated or unsaturated linear, branched or cyclic polyols comprising an alkyl chain and containing from 2 to 16 carbon atoms, in particular from 3 to 8 carbon atoms, bearing, on the alkyl chain, at least two -OH functions, in particular at least three -OH functions, and more particularly at least four -OH functions, and mixtures thereof, and in particular is selected from propylene glycol, 1,3-propanediol, butylene glycol, isoprene glycol, pentylene glycol, hexylene glycol, glycerol, polyglycerols, such as glycerol oligomers, for instance diglycerol, erythritol, arabitol, adonitol, sorbitol, dulcitol, glucose, fructose, xylose, trehalose, sucrose, maltose, saccharose and lactose, and mixtures thereof; polyethylenes glycols, and mixtures thereof.

10. Composition according to any one of the preceding claims, in which said polyol is sorbitol.

11. Composition according to any one of the preceding claims, comprising from 2% to 25% by weight, in particular from 5% to 20% by weight, and more particularly from 6% to 15% by weight of polyol(s), relative to the total weight of the composition.

12. Composition according to any one of the preceding claims, also comprising at least one colorant.

13. Composition according to any one of the preceding claims, said composition being a foundation.

14. Use of at least one volatile linear alkane in an emulsion-type composition comprising a fatty continuous phase, preferably of water-in-oil emulsion type, said composition comprising at least one polyglyceryl polyricinoleate and at least one polyol, for conferring on said composition improved comfort on application and/or improved stability.

15. Cosmetic method for making up and/or caring for keratin materials, comprising at least the application, to said materials, of at least one layer of a composition as defined in any one of Claims I to 13.

## Patentansprüche

1. Kosmetische und/oder dermatologische Zusammensetzung vom Emulsionstyp, umfassend eine zusammenhängende Fettphase, wobei die Zusammensetzung mindestens ein Polyglycerylpolyricinoleat, mindestens ein Polyol und mindestens ein flüchtiges lineares Alkan umfasst.

2. Zusammensetzung gemäß dem vorhergehenden Anspruch, wobei die Zusammensetzung eine Emulsion vom Wasser-in-Öl-Typ ist.

3. Zusammensetzung gemäß Anspruch 1 oder 2, wobei das Alkan 7 bis 17 Kohlenstoffatome und stärker besonders 9 bis 15 Kohlenstoffatome enthält, insbesondere ausgewählt ist aus n-Nonan, n-Decan, n-Undecan, n-Dodecan, n-Tridecan, n-Tetradecan, n-Pentadecan, n-Hexadecan und n-Heptadecan und Gemischen davon.

4. Zusammensetzung gemäß einem der vorhergehenden Ansprüche, wobei das Alkan in der Form eines n-Undecan/n-Tridecan-Gemisches verwendet wird.

5. Zusammensetzung gemäß einem der vorhergehenden Ansprüche, umfassend 1 Gew.-% bis 50 Gew.-%, insbesondere 5 Gew.-% bis 35 Gew.-% und stärker bevorzugt 8 Gew.-% bis 25 Gew.-% flüchtige lineare Alkan(e), relativ zum Gesamtgewicht der Zusammensetzung.

6. Zusammensetzung gemäß einem der vorhergehenden Ansprüche, wobei das Polyglycerylpolyricinoleat resultiert aus der Veresterung von mindestens einem Polyglycerol, das aus den Verbindungen der Formel (I) nachstehend ausgewählt ist: 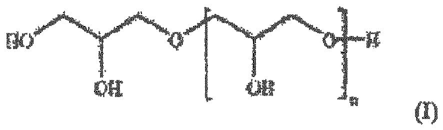 wobei n eine ganze Zahl zwischen 1 und 11 und stärker besonders zwischen 1 und 7 bedeutet; und mindestens einer Polyricinolsäure, die aus den Verbindungen der Formel (II) nachstehend ausgewählt ist: 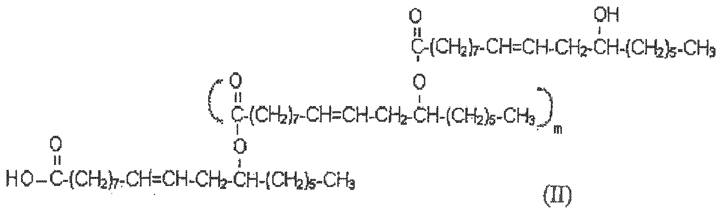 wobei m eine ganze Zahl zwischen 0 rund 10, insbesondere zwischen 1 und 8 und stärker besonders zwischen 1 und 5 bedeutet.

7. Zusammensetzung gemäß einem der vorhergehenden Ansprüche, wobei das Polyglycerylpolyricinoleat aus Polyglyceryl-3-polyricinoleat, Polyglyceryl-5-polyricinoleat, Polyglyceryl-6-polyricinoleat und Polyglyceryl-10-polyricinoleat ausgewählt ist und stärker besonders aus Polyglyceryl-3-polyricinoleat und Polyglyceryl-6-polyricinoleat und Gemischen davon ausgewählt ist.

8. Zusammensetzung gemäß einem der vorhergehenden Ansprüche, umfassend 2 Gew.-% bis 10 Gew.-%, insbesondere 3 Gew.-% bis 8 Gew.-% und stärker besonders 4 Gew.-% bis 7 Gew.-% Polyglycerolpolyricinoleat(e), relativ zum Gesamtgewicht der Zusammensetzung.

9. Zusammensetzung nach einem der vorhergehenden Ansprüche, wobei das Polyol ausgewählt ist aus gesättigten oder ungesättigten linearen, verzweigten oder cyclisehen Polyolen, umfassend eine Alkylkette und enthaltend 2 bis 16 Kohlenstoffatome, insbesondere 3 bis 8 Kohlenstoffatome, tragend an der Alkylkette mindestens zwei Funktionen -OH, insbesondere mindestens drei Funktionen -OH und stärker besonders mindestens vier Funktionen -OH, und Gemischen davon, und insbesondere ausgewählt ist aus Propylenglycol, 1,3-Propandiol, Butylenglycol, Isoprenglycol, Pentylenglycol, Hexylenglycol, Glycerol, Polyglycerolen, wie Glycerololigomeren, zum Beispiel Diglycerol, Erythritol, Arabitol, Adonitol, Sorbitol, Dulcitol, Glucose, Fructose, Xylose, Trehalose, Sucrose, Maltose, Saccharose und Lactose, und Gemischen davon; Polyethylenglycolen, und Gemischen davon.

10. Zusammensetzung gemäß einem der vorhergehenden Ansprüche, wobei das Polyol Sorbitol ist.

11. Zusammensetzung gemäß einem der vorhergehenden Ansprüche, umfassend 2 Gew.-% bis 25 Gew.-%, insbesondere 5 Gew.-% bis 20 Gew.-% und stärker besonders 6 Gew.-% bis 15 Gew.-% Polyol(e), relativ zum Gesamtgewicht der Zusammensetzung.

12. Zusammensetzung gemäß einem der vorhergehenden Ansprüche, auch umfassend mindestens ein farbgebendes Mittel.

13. Zusammensetzung gemäß einem der vorhergehenden Ansprüche, wobei die Zusammensetzung eine Grundlage ist.

14. Verwendung von mindestens einem flüchtigen linearen Alkan in einer Zusammensetzung von Emulsionstyp, umfassend eine zusammenhängende Fettphase, bevorzugt vom Wasser-in-Öl-Emulsionstyp, wobei die Zusammensetzung mindestens ein Polyglycerylpolyrinoleat und mindestens ein Polyol umfasst, um der Zusammensetzung verbesserten Verwendungskomfort und/oder verbesserte Stabilität zu verleihen.

15. Kosmetisches Verfahren zum Schminken und/oder Pflegen von Keratinmaterialien, umfassend mindestens das Aufbringen mindestens einer Schicht einer Zusammensetzung wie in einem der Ansprüche 1 bis 13 definiert auf die Materialien.

## Revendications

1. Composition cosmétique et/ou dermatologique de type émulsion comprenant une phase continue grasse, ladite composition comprenant au moins un polyricinoléate de polyglycéryle, au moins un polyol et au moins un alcane linéaire volatil.

2. Composition selon la revendication précédente, ladite composition étant de type émulsion eau-dans-huile.

3. Composition selon la revendication 1 ou 2, dans laquelle ledit alcane comprend de 7 à 17 atomes de carbone, et plus particulièrement de 9 à 15 atomes de carbone, et en particulier est choisi parmi le n-nonane, le n-décane, le n-undécane, le n-dodécane, le n-tridécane, le n-tétradécane, le n-pentadécane, le n-hexadécane, le n-heptadécane, et leurs mélanges.

4. Composition selon l'une quelconque des revendications précédentes, dans laquelle ledit alcane est utilisé sous la forme d'un mélange n-undécane/n-tridécane.

5. Composition selon l'une quelconque des revendications précédentes, comprenant de 1 % à 50 % en poids, en particulier de 5 % à 35 % en poids, et plus préférentiellement de 8 % à 25 % en poids d'alcane(s) linéaire(s) volatil(s) par rapport au poids total de la composition.

6. Composition selon l'une quelconque des revendications précédentes, dans laquelle le polyricinoléate de polyglycéryle résulte de l'estérification d'au moins un polyglycérol choisi parmi les composés de formule (I) ci-dessous : 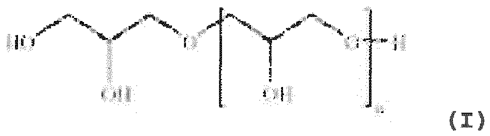 dans laquelle n désigne un entier compris entre 1 et 11 et plus particulièrement entre 1 et 7 ;
et d'au moins un poly(acide ricinoléique) choisi parmi les composés de formule (II) ci-dessous : 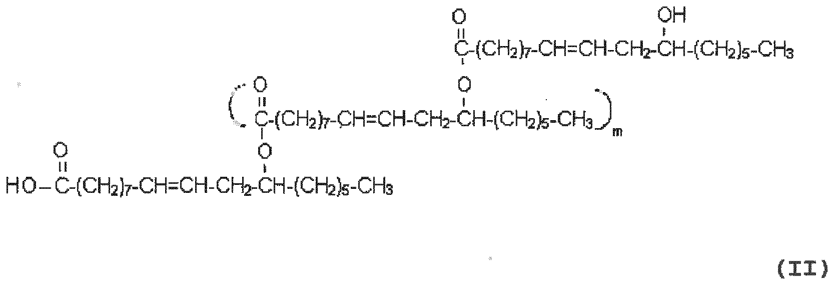 dans laquelle m désigne un entier compris entre 0 et 10, en particulier entre 1 et 8 et plus particulièrement entre 1 et 5.

7. Composition selon l'une quelconque des revendications précédentes, dans laquelle ledit polyricinoléate de polyglycéryle est choisi parmi le polyricinoléate de polyglycéryle-3, le polyricinoléate de polyglycéryle-5, le polyricinoléate de polyglycéryle-6, et le polyricinoléate de polyglycéryle-10, et plus particulièrement est choisi parmi le polyricinoléate de polyglycéryle-3 et le polyricinoléate de polyglycéryle-6, et leurs mélanges.

8. Composition selon l'une quelconque des revendications précédentes, comprenant de 2 % à 10 % en poids, en particulier de 3 % à 8 % en poids et plus particulièrement de 4 % à 7 % en poids de polyricinoléate(s) de polyglycérol par rapport au poids total de la composition.

9. Composition selon l'une quelconque des revendications précédentes, dans laquelle ledit polyol est choisi parmi les polyols saturés ou insaturés linéaires, ramifiés ou cycliques, comprenant une chaîne alkyle et contenant de 2 à 16 atomes de carbone, en particulier de 3 à 8 atomes de carbone, portant sur la chaîne alkyle au moins deux fonctions -OH, en particulier au moins trois fonctions -OH et plus particulièrement au moins quatre fonctions -OH, et leurs mélanges, et en particulier est choisi parmi le propylèneglycol, le 1,3-propanediol, le butylèneglycol, l'isoprèneglycol, le pentylèneglycol, l'hexylèneglycol, le glycérol, les polyglycérols, tels que les oligomères de glycérol, par exemple diglycérol, l'érythritol, l'arabitol, l'adonitol, le sorbitol, le dulcitol, le glucose, le fructose, le xylose, le tréhalose, le sucrose, le maltose, le saccharose et le lactose et leurs mélanges ; les polyéthylèneglycols, et leurs mélanges.

10. Composition selon l'une quelconque des revendications précédentes, dans laquelle ledit polyol est le sorbitol.

11. Composition selon l'une quelconque des revendications précédentes, comprenant de 2 % à 25 % en poids, en particulier de 5 % à 20 % en poids, et plus particulièrement de 6 % à 15 % en poids de polyol(s) par rapport au poids total de la composition.

12. Composition selon l'une quelconque des revendications précédentes, comprenant en outre au moins un colorant.

13. Composition selon l'une quelconque des revendications précédentes, ladite composition étant un fond de teint.

14. Utilisation d'au moins un alcane linéaire volatil dans une composition de type émulsion comprenant une phase continue grasse, de préférence de type émulsion eau-dans-huile, ladite composition comprenant au moins un polyricinoléate de polyglycéryle et au moins un polyol, pour conférer à ladite composition un confort à l'application amélioré et/ou une stabilité améliorée.

15. Procédé cosmétique de maquillage et/ou de soin des matières kératiniques, comprenant au moins l'application sur lesdites matières d'au moins une couche d'une composition telle que définie selon l'une quelconque des revendications 1 à 13.
